# EUROPÄISCHE PATENTSCHRIFT

(11) **EP 0 626 950 B1**
(45) Veröffentlichungstag und Bekanntmachung des Hinweises auf die Patenterteilung: **24.10.2001**
(21) Anmeldenummer: 93903896.4
(22) Anmeldetag: 01.02.1993
(51) Int. Cl.: C07D 251/70, A61K 7/42, C07D 251/34, C07D 251/46, C07D 251/52

(54) **TRIAZIN-DERIVATE MIT UV-FILTER EIGENSCHAFTEN**
TRIAZINE DERIVATES WITH UV-FILTER PROPERTIES
DERIVES DE TRIAZINE A PROPRIETES DE FILTRAGE DES RAYONS UV

(30) Priorität: 19.02.1992 DE 4204923; 21.07.1992 DE 4223890
(43) Veröffentlichungstag der Anmeldung: 07.12.1994
(73) Patentinhaber: MERCK PATENT GmbH, 64271 Darmstadt (DE)
(72) Erfinder: STEIN, Ingeborg, D-6106 Erzhausen (DE); CASUTT, Michael, D-6148 Heppenheim (DE); HEYWANG, Ulrich, D-6100 Darmstadt (DE); MARTIN, Roland, D-6940 Weinheim (DE); SCHWARZ, Michael, W., D-6080 Gro -Gerau (DE)
(86) Internationale Anmeldenummer: EP9300226
(87) Internationale Veröffentlichungsnummer: WO9317002

(56) Entgegenhaltungen:
- EP-A- 0 087 098
- EP-A- 0 457 687
- EP-A- 0 507 692
- US-A- 4 061 730

## Beschreibung

Die Erfindung betrifft Triazin-Derivate der Formel I 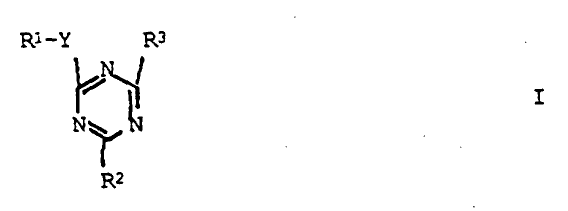 wobei
- Y: NH oder O,
- R¹: einen Rest der Formel II 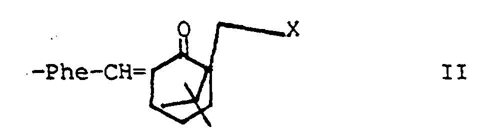
worin
- Phe: eine unsubstituierte oder durch 1 bis 4 Alkyl- oder Alkoxygruppen mit 1 bis 10 C-Atomen substituierte Phenylengruppe, und
- X: H oder -SO₃H
bedeuten, und
- R² und R³: jeweils unabhängig voneinander einen Rest der Formel Y-R¹,
oder
Alkoxy, Alkylamino mit 1 bis 10 C-Atomen, Arylamino oder Aryloxy mit bis zu 10 C-Atomen
bedeuten, wobei jedoch Verbindungen ausgenommen sind, in denen jeweils zwei der Triazin-Substituenten Y-R¹, R² oder R³ 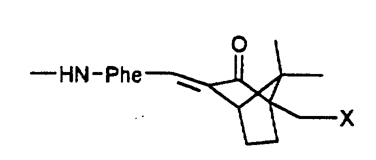 mit
- Phe: unsubstituierte Phenylengruppe
- X: H
und der dritte Triazin-Substituent
Alkoxy oder Alkylamino mit 8 bis 10 C-Atomen bedeuten, sowie Verfahren zu ihrer Herstellung und ihre Verwendung in kosmetischen Zubereitungen, insbesondere zum Schutz vor Sonnenstrahlung, und in pharmazeutischen Zubereitungen zur vorbeugenden Behandlung von Entzündungen und Allergien der Haut oder bestimmter Krebsarten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen:
sie bewirken, daß die Haut ihre Elastizität verliert und sich Falten bilden und führen somit zu einer frühzeitigen Alterung. Manchmal kann man auch Dermatosen beobachten.

Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbieren können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet:

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf. Dagegen sind nur wenige wasserlösliche UVA-Filter derzeit bekannt, deren UV-Absorption jedoch gering ist.

Aus der DE 34 08 406 sind ähnliche Triazin-Derivate bekannt. Diese weisen jedoch keine Methylencamphylidengruppe am Phenylenring auf. Diese Verbindungen können zwar auch als UV-Filter in Sonnenschutzmitteln verwendet werden, weisen jedoch keine Absorption im UVA-Bereich auf. Weiterhin sind diese Verbindungen in herkömmlichen kosmetischen Trägern, insbesondere in wäßrigen Suspensionen, nur begrenzt löslich, so daß sie in Sonnenschutzmitteln meist zusammen mit anderen UV-Filtern eingesetzt werden müssen.

In EP 0 507 692 A1 wiederum werden Triazin-Derivate beschrieben, welche zweifach durch Aminobenzylidencampher-Reste substituiert sind. Diese Triazinderivate werden unter anderem verwendet, um kosmetische Formulierungen zum Schutz der Haut und der Haare gegen UV-Strahlung herzustellen.

In der deutschen Auslegeschrift DE 12 05 970 werden Triazin-Derivate zum Schutz organischer Stoffe vor UV-Strahlung beschrieben, worin die Triazingruppe mit einer Benzooxazolylanilinogruppe verknüpft ist. Diese Verbindungen absorbieren UV-Strahlen mit hohen Wellenlängen, z.B. von 370-380 nm und sind nicht als Bestandteile kosmetischer Zusammensetzungen geeignet.

Es wurde gefunden, daß (4-Camphylidenmethylenanilino bzw. -phenoxy)-1,3,5-Triazin-Derivate, insbesondere 1,3,5-Triazine, die in 2,4- oder in 2,4,6-Stellung mit Aminoarylidencampher verknüpft sind, hervorragende UVA-Filter-Eigenschaften besitzen. Ihre Löslichkeit in den in der Kosmetik verwandten Ölen ist sehr gut, so daß Einsatzkonzentrationen auch in komplizierten Formulierungen bis mindestens 10 % der Zubereitung möglich sind. Eine wasserlösliche Form des neuen Filters sind Sulfonsäure-Derivate (X = SO₃H). Die Wasserlöslichkeit ist hier so gut, daß ebenfalls Einsatzkonzentrationen von 10 % möglich sind.

Weiterhin weisen die erfindungsmäßigen Verbindungen eine außerordentliche Photostabilität gegenüber UV-Strahlung auf, die die Stabilität bisher bekannter UV-Filtersubstanzen bei weitem übersteigt.

Falls die Extinktion im UVB-Bereich ein Minimum aufweist, ist dies kein Nachteil, da man problemlos einen UVB-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung sind die Verbindungen der oben gegebenen Formel I, insbesondere worin Phe 1,4-Phenylen bedeutet.

Bevorzugte Ausführungsformen der vorliegenden Erfindung sind:
a) Triazin-Derivate der Formel I1 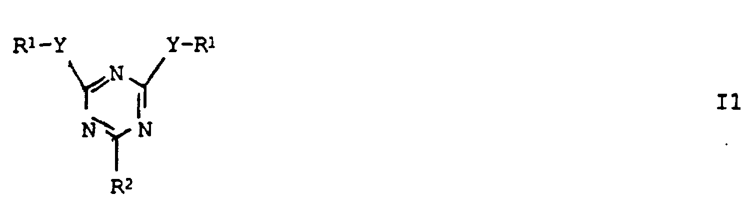 worin R¹, R² und Y die angegebene Bedeutung besitzen;
b) Triazin-Derivate der Formel I2 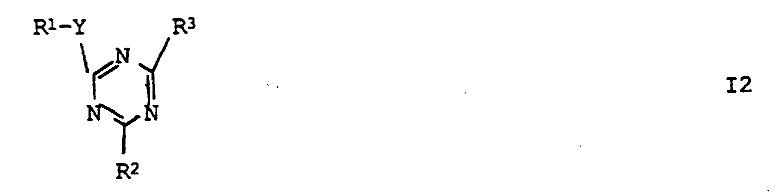 worin
   - R¹ und Y: die angegebene Bedeutung besitzen, und
   - R² und R³: jeweils unabhängig voneinander Alkoxy, Alkylamino, Arylamino mit 1 bis 10 C-Atomen oder Aryloxy mit bis zu 10 C-Atomen bedeuten;
c) Triazin-Derivate der Formel Ia, 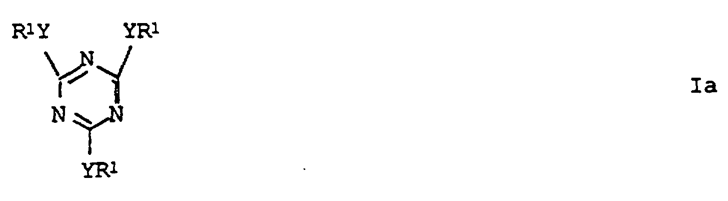 worin Phe eine unsubstituierte 1,4-Phenylengruppe und X H bedeutet.
d) Triazin-Derivate der Formeln I, I1, I2 und Ia, worin Y NH - bedeutet;
e) Triazin-Derivate der Formel Ic, 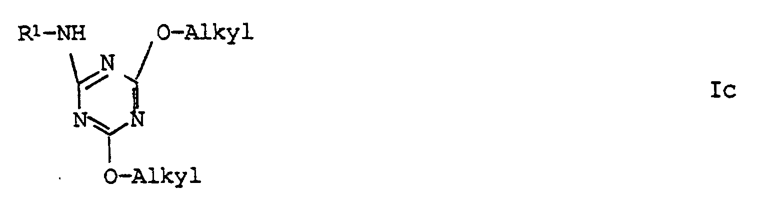 worin R¹ die angegebene Bedeutung besitzt, und
   Alkyl Alkyl mit 1 bis 10 C-Atomen bedeutet;
f) Triazin-Derivate der Formel Id, 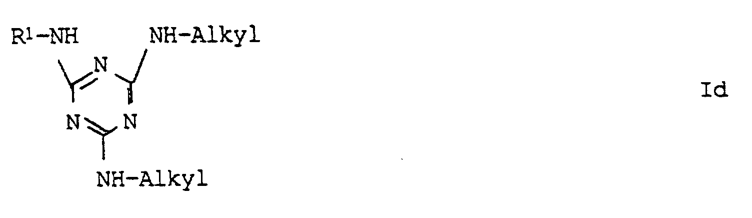 worin R¹ die angegebene Bedeutung besitzt, und Alkyl Alkyl mit 1 bis 10 C-Atomen bedeutet.
g) Triazin-Derivate der Formel I,
   worin die Reste R² und R³ Alkylamino bzw. Anilino bedeuten,
   - Y: NH ist und Phe eine durch 1 bis 4, vorzugsweise 1 oder 2, Alkyl- oder Alkoxygruppen substituierte Phenylengruppe oder eine 1,3-Phenylengruppe bedeutet;
h) Triazin-Derivate der Formel I, worin R² und R³ einen Rest der Formel Y-R¹ bedeuten;
i) Triazin-Derivate der Formel I,
   worin R² einen Rest der Formel Y-R¹ bedeutet und R³ Alkylamino ist, wobei die 2-Ethylhexylaminogruppe ausgenommen ist;
j) Triazin-Derivate der Formel I
   worin X SO₃H bedeutet.

Die Phenylengruppe Phe ist vorzugsweise eine unsubstituierte oder durch eine bis vier Alkyl- oder Alkoxygruppen substituierte 1,4-Phenylengruppe.

Vorzugsweise ist die Phenylengruppe unsubstituiert oder substituiert mit einer oder zwei Alkoxygruppen mit 1 bis 8 C-Atomen, insbesondere mit Methoxy, Ethoxy- oder 2-Ethylhexyloxygruppen.

Sowohl im vorstehenden als auch im nachstehenden Text versteht es sich von selbst, daß von all den in Frage kommenden Verbindungenen solche ausgenommen sind, in denen jeweils zwei der Triazin-Substituenten, die den in der allgemeinen Formel I mit Y-R¹, R² oder R³ bezeichneten Resten entsprechen, 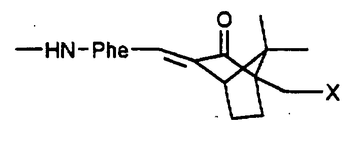 mit
- Phe: unsubstituierte Phenylengruppe
- X: H
bedeuten und in denen der dritte Triazin-Substituent
Alkoxy oder Alkylamino mit 8 bis 10 C-Atomen
bedeutet.

Bevorzugte Verbindungen der Formel I sind diejenigen der Formeln I3 bis I18, wobei A eine Gruppe der Formel 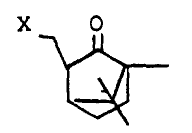
- R⁴: Alkyl oder Alkoxy mit 1 bis 10 C-Atomen bedeutet und n 1 oder 2 bedeutet.

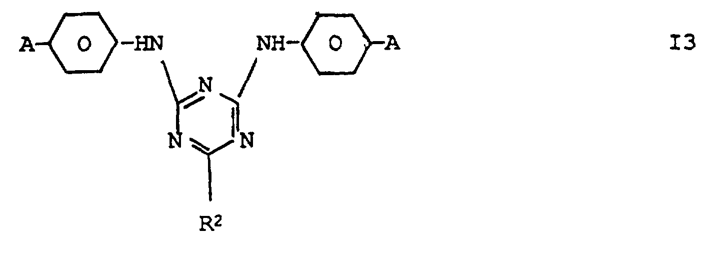

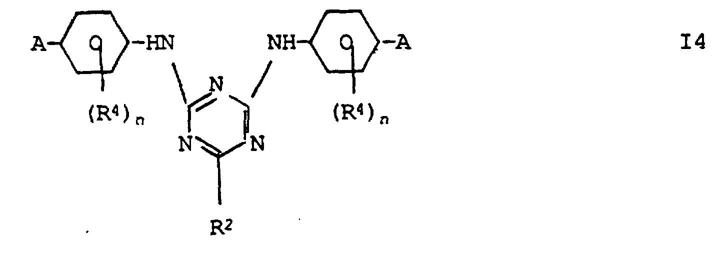

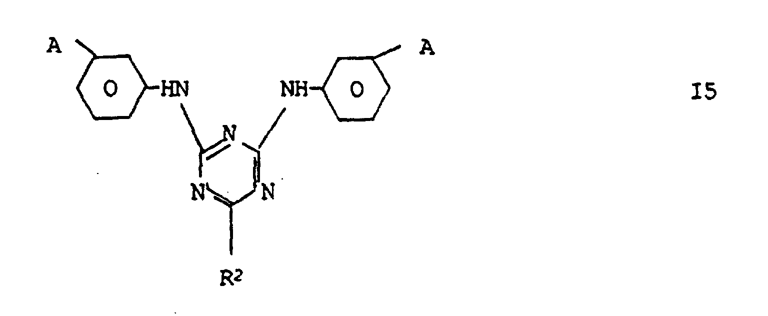

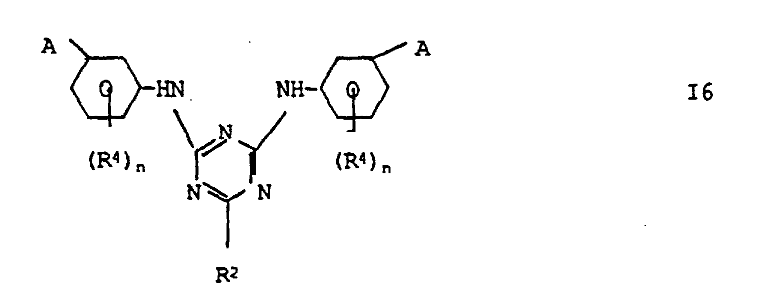

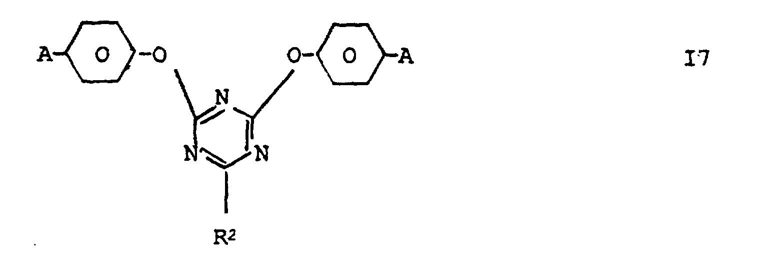

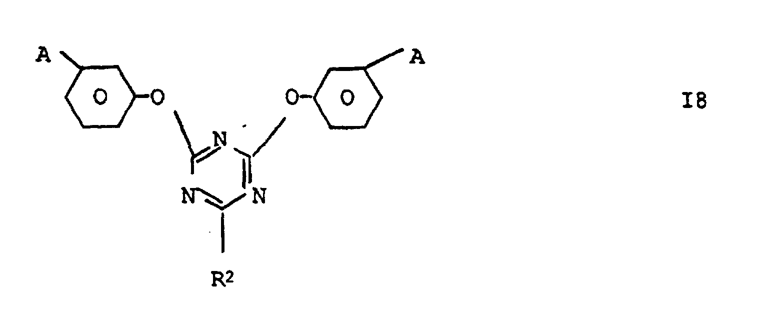

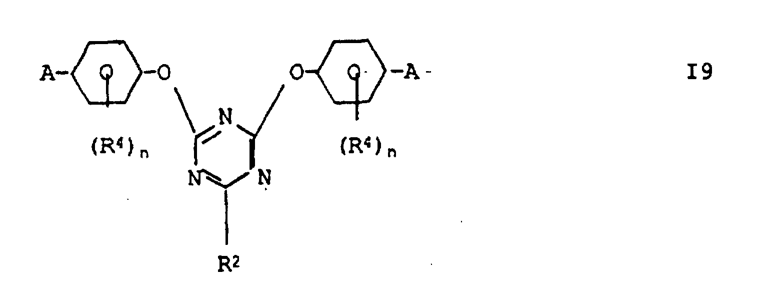

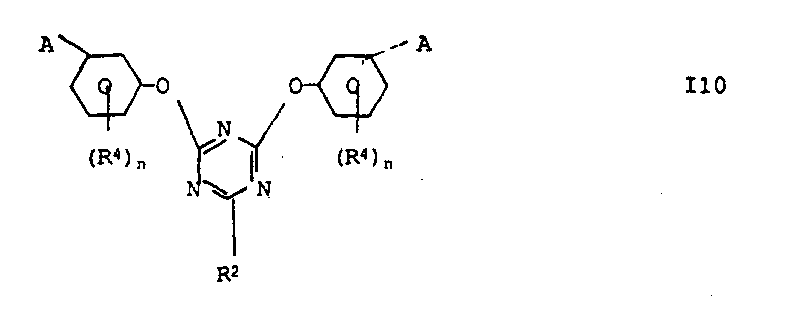

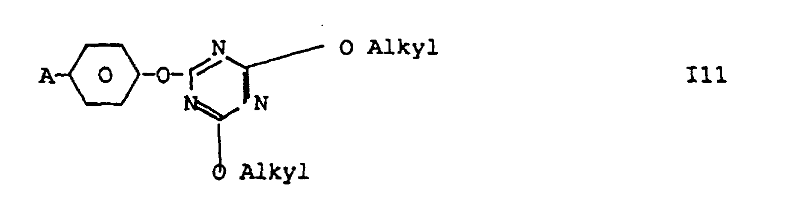

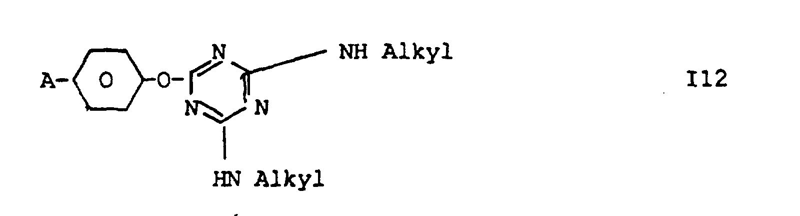

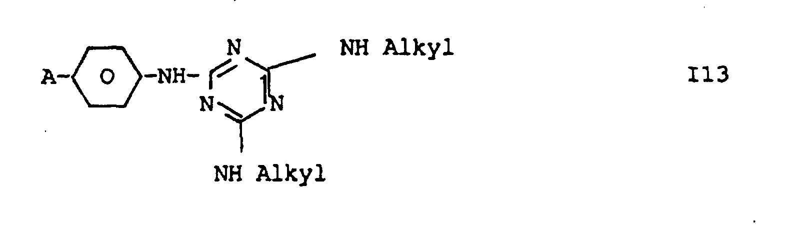

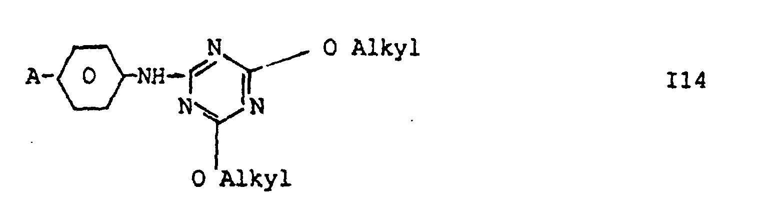

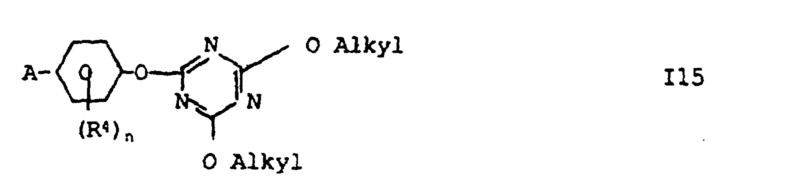

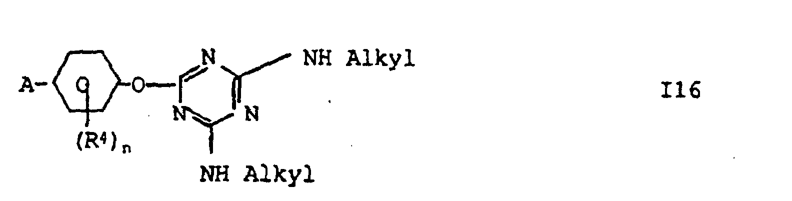

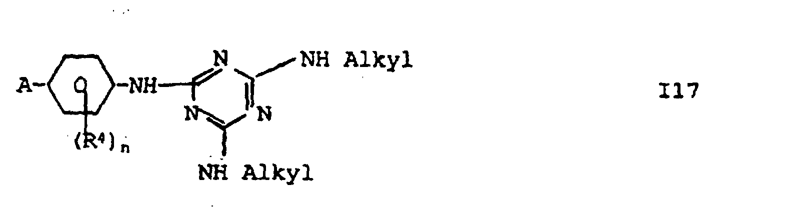

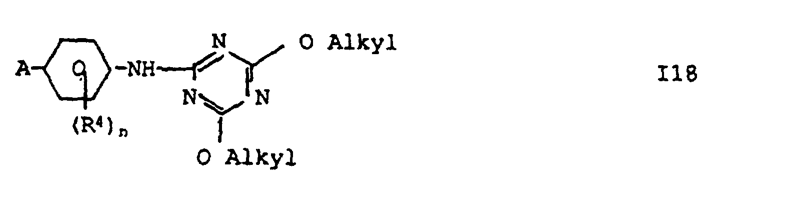

In der bevorzugten Verbindungen der Formel I3 bis I18 weist R² vorzugsweise dieselbe Bedeutung wie die Substituenten in der 2- und 6-Position auf oder bedeutet einen Alkoxy, Alkylamino mit 1 bis 10 C-Atome Arylamino oder Aryloxyrest mit bis zu 10 C-Atomen, insbesondere Methoxy, Ethoxy, 2-Ethylhexyloxy, Methylamino, Ethylamino, 2-Ethylhexylamino, Anilino-, p-Alkylanilino, O-Alkylanilino, Phenoxy, p-Alkylphenoxy, o-Alkylphenoxy.

Man erhält die Verbindungen der Formel I z.B. dadurch, daß man ein Amino- bzw. Hydroxybenzylidencampher-Derivat der Formel II,

HY-Phe-A II

worin Phe, A und Y die angegebene Bedeutung besitzen, mit Cyanurchlorid umsetzt.

Setzt man dabei ca. 3 Mol der Verbindung der Formel II mit einem Mol Cyanurchlorid bei Temperaturen zwischen 20 °C und 150 °C, vorzugsweise unter Rückfluß in einem inerten Lösungsmittel um, erhält man die Verbindung der Formel I, worin R² und R³ einen Rest der Formel R¹-Y bedeuten.

Wird die Reaktion bei niedrigen Temperaturen, d.h. zwischen -40 °C und 80 °C, vorzugsweise 0 °C und 60 °C, insbesondere zwischen 0 °C und Raumtemperatur, gegebenenfalls in Gegenwart einer schwachen Base, mit nur ca. 1 Mol an Verbindung der Formel II, bezogen auf 1 Mol Cyanurchlorid, durchgeführt, erhält man intermediär ein Triazin-Derivat der Formel III, 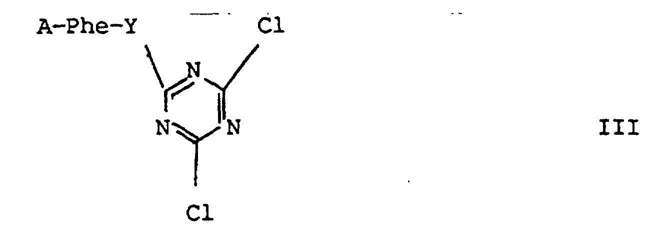 worin A, Phe und Y die angegebene Bedeutung besitzen.

Bei Umsetzung von ca. 2 Mol an Verbindung der Formel II mit Cyanurchlorid bei diesen Temperaturen erhält man intermediär ein Triazinderivat der Formel IV 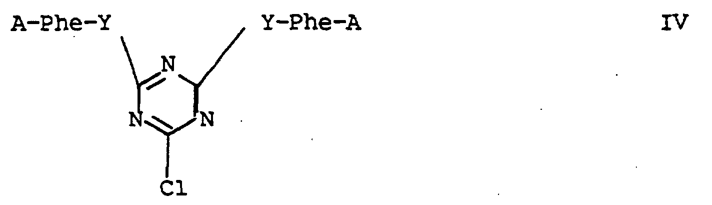 worin A, Phe und Y die angegebene Bedeutung besitzen.

Die Verbindungen der Formel III und IV sind neu und ebenfalls Gegenstand der Erfindung.

Anschließend wird die Verbindung der Formel III bzw. IV mit einem weiteren Equivalent einer Verbindung der Formel II, worin Y, Phe und/oder A von der ursprünglich eingesetzten Verbindung der Formel II verschieden sind, oder mit einem Alkohol, Alkylamin mit 1 bis 10 C-Atomen, Anilin oder Phenol mit bis zu 10 C-Atomen umgesetzt.

Bei Temperaturen zwischen 0°C und Raumtemperatur lassen sich insbesondere Aminoverbindungen der Formel IIa,

R¹NH₂ IIa

worin R¹ die angegebene Bedeutung besitzt, gegebenenfalls in Gegenwart einer schwachen Base, mit Cyanurchlorid umsetzen, wobei das Triazin-Derivat der Formel IIIa, 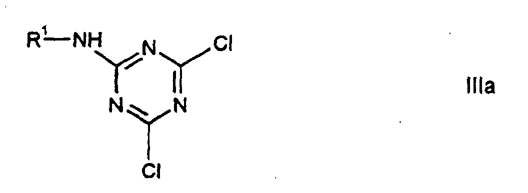 worin R¹ die angegebene Bedeutung besitzt,entsteht. Zur Herstellung entsprechender Verbindungen der Formel I wird es isoliert und anschließend mit mindestens 2 Mol eines Alkohols umgesetzt.

Weiterhin können die Verbindungen der Formel I, worin R² von R¹-Y verschieden ist, hergestellt werden, indem man ein Mol eines Alkohols, Alkylamins, Anilins oder Phenols der Formel CₙH₂ₙ₊₁-(Phe)-Y-H, worin o 0 oder 1, n 1 bis 10, im Falle o = 1, 0-4 bedeutet, in Gegenwart einer Base mit einem Mol Cyanurchlorid bei Temperaturen zwischen -40 °C und +40 °C umsetzt, und anschließend die so erhaltene Verbindung der Formel V 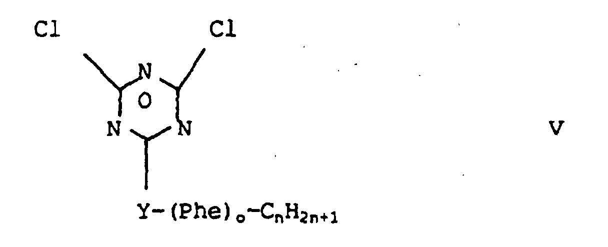 mit 2 Equivalenten einer Verbindung der Formel II umsetzt.

Die Verbindungen der Formel II sind entweder bekannt oder können nach an sich bekannten Methoden hergestellt werden, z.B. durch Kondensation eines Amino- oder Hydroxybenzaldehyds mit Campher bzw. Camphersulfonsäure, z.B. nach einer in DE 40 27 980 beschriebenen Methode.

Gegenstand der Erfindung ist auch das Verfahren zur Herstellung der neuen Verbindungen der Formel I.

Ein weiterer Gegenstand der Erfindung ist eine kosmetische Zubereitung, welche in einem kosmetisch verträglichen Träger eine wirksame Menge mindestens eines Derivates der obigen Formel I enthält.

Insbesondere bevorzugt sind solche kosmetischen Zubereitungen, worin der Träger mindestens eine Fettphase aufweist und in der Verbindung der Formel I X H bedeutet, bzw. solche, worin der Träger mindestens eine wäßrige Phase aufweist und X SO₃H bedeutet.

Das erfindungsgemäße kosmetische Mittel kann als Mittel zum Schutz der menschlichen Epidermis oder der Haare oder als Sonnenschutzmittel verwendet werden.

Gegenstand der Erfindung ist ferner ein Verfahren zum Schutz der Haut und natürlicher oder sensibilisierter Haare von Sonnenstrahlen, wobei auf die Haut oder die Haare eine wirksame Menge mindestens einer Verbindung der Formel I aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Benzylidencampher-Derivates der obigen Formel I umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, öligwäßriger oder wäßrig-alkoholischer Gele oder als feste Stifte vorliegen oder als Aerosol konfektioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 0,1 bis 10 Gew.%, vorzugsweise 0,3 bis 6 Gew.% bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel I enthalten und andere UVB- und/oder UVA-Filter umfassen können.

In diesem Fall beträgt die Menge des Filters der Formel I in der Regel zwischen 0,2 und 8,0 Gew.%, vorzugsweise zwischen 0,4 und 5,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 0,3 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Haarprodukte, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (Fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können. Derartige Mittel enthalten in der Regel 0,3 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 280 bis 400 nm, insbesondere die der Formel Ia in einem Bereich von 320 bis 400 nm und die der Formel Ib in einem Bereich von 280 bis 350 nm.

Ein weiterer Gegenstand der Erfindung ist die Verwendung der Verbindungen der Formel I als kosmetische Produkte.

Wie oben bereits erwähnt, hat die Anmelderin im Laufe ihrer Untersuchungen außerdem festgestellt, daß die Verbindungen der Formel I eine signifikante pharmakologische Aktivität auf dem Gebiet der Präventivbehandlung von Entzündungen und Hautallergien zeigen.

Gegenstand der Erfindung ist daher auch die Verwendung der Verbindungen der Formel I zur Herstellung eines Medikaments.

Gegenstand der Erfindung ist ferner ein pharmazeutisches Mittel, welches eine wirksame Menge mindestens einer Verbindung der Formel I als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nicht-steroide antiinflammatorische Mittel, Carotinoide und Mittel gegen Psoriasis.

Dieses Mittel kann auch Geschmacksverbesserungsmittel, Konservierungsmittel, Stabilisatoren, Feuchtigkeitsregulatoren, pH-Regulatoren, Modifikatoren für den osmotischen Druck, Emulgatoren, Lokalanaesthetika, Puffer etc. enthalten.

Es kann außerdem in an sich bekannter Weise in Retard-Form oder in einer Form konditioniert sein, in welcher der Wirkstoff rasch freigesetzt wird.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen, sowie der korrespondierenden Anmeldungen P 42 04 923, eingereicht am 19.02.1992 und P 42 23 890, eingereicht am 21.07.1992, sind durch Bezugnahme in diese Anmeldung eingeführt.

Die nachfolgenden Beispiele sind repräsentativ für die vorliegende Erfindung.

Extinktion werden bei der angegebenen Wellenlänge bei einer Schichtdicke von 1 cm bestimmt.

### Beispiel 1

### 2,4,6-Tris(4-camphylidenmethylen-anilino)-1,3,5-triazin

Zu einer Lösung von 25,5 g (0,1 Mol) 4-Amino-benzylidencampher in 500 ml Toluol werden bei 0 °C 6 g (33 mMol) Cyanurchlorid in 200 ml Toluol zugetropft. Die Reaktionsmischung wird unter Rückfluß erhitzt. Nach beendeter Reaktion wird der ausgefallene weiße Feststoff abgesaugt, nachgewaschen und ausgerührt. Fp.: 336 °C. Die weiteren Spektren entsprechen der erwarteten Verbindung.
UV (Ethanol, c = 1 mg/100 ml) : λₘₐₓ = 354 nm, E = 1,2

Analog werden hergestellt:
2,4,6-Tris[3-camphylidenmethylen-anilino]-1,3,5-triazin
2, 4, 6-Tris [4- (camphylidenmethylen) -2-methoxyanilino]-1,3,5-triazin
2,4,6-Tris[3-(camphylidenmethylen) -2-methoxyanilino]-1,3,5-triazin
2, 4, 6-Tris[4-(camphylidenmethylen)-3-methoxyanilino]-1, 3, 5-triazin
2,4, 6-Tris [4-(camphylidenmethylen)-3- (2-ethylhexyloxy)-anilino]-1,3,5-triazin

### Beispiel 2

### 2,4,6-Tris(camphyliden-methylenphenyl-4-oxy)-1,3,5-triazin

7,7 g (30 mmol 2-(4-Hydroxy-benzyliden)-campher in 100 ml einer 1 molaren NaOH-Lösung werden zu einer Lösung von 1,8 g (10 mmol) Cyanurchlorid in 100 ml Methylenchlorid getropft und unter Rückfluß erhitzt. Nach beendeter Reaktion wird die organische Phase abgetrennt und mit Wasser gewaschen. Die entstandenen weißen Kristalle zeigen die erwarteten Spektren.
UV (Ethanol, c = 1 mg/100 ml) : λₘₐₓ = 295 nm, E = 0,98

Analog werden hergestellt:
2,4,6-Tris[4-(camphylidenmethylen)-3-methoxyphenoxy]-1,3,5-triazin
2,4,6-Tris[3-(camphylidenmethylen)-phenoxy]-1,3,5-triazin
2,4,6-Tris[4-(camphylidenmethylen)-2-methoxyphenoxy]-1,3,5-triazin

### Beispiel 3

### 2,4-Bis (2-ethyl-hexylamino)-6-(4-camphylidenmethylen-anilino) -1,3,5-triazin

a) 2-(4-Camphylidenmethylen-anilino)-4,6-dichloro-1,3,5-triazin
   Zu einer Lösung von 0,1 Mol Cyanurchlorid in Toluol wird 0.1 Mol 4-Aminobenzylidencampher, gelöst in Toluol, zugetropft. Das Reaktionsgemisch wird auf 40 °C erwärmt. Die Aufarbeitung führt zu 36 g (88 %) eines hellgelben Feststoffes. Fp.: 238 °C.
b) 2, 4-Bis (2-ethyl-hexylamino) -6- (4-camphylidenmethylen-anilino)-1,3,5-triazin
   Ein Reaktionsgemisch bestehend aus 33 g (0.26 Mol) 2-Ethylhexylamin und 50 g (0.12 Mol) 2-(4-Camphylidenmethylen-anilino)-4,6-dichloro-1,3,5-triazin in Toluol wird in Gegenwart von Natriumacetat auf 90 °C erhitzt. Nach vollständiger Umsetzung der Edukte wird aufgearbeitet. Das Produkt (95 %) ist ein gelbes zähflüssiges Öl. UV (Ethanol, c = 1 mg/100 ml) : λₘₐₓ = 345 nm, E = 0.57.

### Beispiel 4

### 2,4-Bis-ethoxy-6-(4-camphylidenmethylen-anilino)-1,3,5-triazin

Zu einer Lösung von 0.3 Mol Natriumethanolat in Ethanol werden 0.05 Mol 2-(4-Camphylidenmethylen-anilino)-4,6-dich-loro-1,3,5-triazin (siehe Beispiel 3 a) zugegeben und auf 60 °C erhitzt. Nach Aufarbeitung wird das Produkt als weiße Kristalle (Fp.: 161 °C) in 63 % Ausbeute erhalten.
UV (Ethanol, c = 1 mg/100 ml) : λₘₐₓ = 335 nm, E = 0.90.

### Beispiel 5

### 2,4-Bis(2-ethyl-hexyloxy)-6-(4-camphylidenmethylen-anilino) -1, 3, 5-triazin

a) 2,4-Bis(2-ethyl-hexyloxy)-6-chloro-1,3,5-trizin
   Durch Zutropfen von 47 ml (0.3 Mol) 2-Ethyl-hexanol zu einer Suspension von 0.3 Mol Kaliumhydrid in Tetrahydrofuran und anschließendes Erhitzen wird das Kaliumsalz des Alkohols dargestellt. Die Lösung wird bei 0 °C zu einer Lösung von Cyanurchlorid (0.13 Mol) in Tetrahydrofuran langsam zugetropft. Nachdem zwei Stunden bei 20 °C nachgerührt wurde, wird aufgearbeitet. Die Destillation führt zu 23 g (41 %) reinem Produkt. Siedepunkt = 181 °C/0,022 torr.
b) 2,4-Bis(2-ethyl-hexyloxy)-6-(4-camphylidenmethylen-anilino)-1,3,5-triazin
   Zu einer Lösung von 0.1 Mol 2,4-Bis(2-ethyl-hexyloxy)-6-chloro-1,3,5-triazin in Toluol wird 0.1 Mol 4-Aminobenyliden-campher zugegeben und bis zur vollständigen Umsetzung auf 90 °C erhitzt. Die Aufarbeitung führt zu 55 g (94 %) eines gelben zähflüssigen Öls.
   UV (Ethanol, c = 1 mg/100 ml) : λₘₐₓ = 334 nm, E = 0.64. Analog werden hergestellt:
   2,4-Bis(phenoxy)-6-(4-camphylidenmethylen-anilino)-1,3,5-triazin, UV (Ethanol, c = 1 m/100 ml): λₘₐₓ = 335 nm,
   E = 0.71, Fₚ: 207 °C.

### Beispiel 6

### 2,4-Bis(4-Camphylidenmethylen-anilino)-6-ethoxy-1,3,5-triazin

a) 2,4-Bis(4-Camphylidenmethylen-anilino)-6-chloro-1,3,5-triazin
   Zu einer Lösung von Cyanurchlorid in Toluol wird 4-Aminobenzyliden-campher (Molverhältnis 1:2) ebenfalls in Toluol langsam zugetropft und unter DC-Kontrolle auf 60 °C erwärmt. Nach der Aufarbeitung wird zu 45 % die oben genannte Verbindung erhalten. Fp.: 221 °C.
b) 2,4-Bis(4-Camphylidenmethylen-anilino)-6-ethoxy-1,3,5-triazin
   2,4-Bis (4-Camphylidenmethylen-anilino) -6-chloro-1,3,5-triazin wird analog der Vorschrift von Beispiel 2 mit Natriumethanolat umgesetzt. Es entsteht zu 43 % das gewünschte Produkt. UV (Ethanol, c = 1 mg/100 ml) : λₘₐₓ = 346 nm, E = 0.95, Fp.: 198 °C

### Beispiel 7

### 2,4-Bis(4-Camphylidenmethylen-anilino)-6-propyloxy-1, 3-5-triazin

Zu einem Gemisch von 0.1 Mol 2,4-Dichloro-6-propyloxy-1,3,5-triazin in Toluol wird eine Lösung von 0.2 Mol 4-Aminobenzyliden-campher in Toluol zugegeben und auf 94 °C erhitzt. Die Aufarbeitung führt zu 31 g (48 %) weißer Kristalle. Fp.: 164 °C.
UV (Ethanol, c = 1 mg/100 ml) : λₘₐₓ = 345 nm, E = 0.94

Die folgenden Verbindungen wurden analog der Beispiele 6 und 7 synthetisiert:
2,4-Bis(4-Camphylidenmethylen-anilino)-6-butyloxy-1,3,5-triazin
UV (Ethanol, c = 1 mg/100 ml) : λₘₐₓ = 345 nm, E = 0.88, Fp.: 181 °C.

2,4-Bis(4-Camphylidenmethylen-anilino)-6-phenoxy-1,3,5-triazin
UV (Ethanol, c = 1 mg/100 ml) : λₘₐₓ = 348 nm, E = 0.95, Fp.: 185 °C.

### Beispiel 8

### Sonnenschutzcreme (W/O)

| % | | | |
|---|---|---|---|
| A | 2,4,6-Tris(4-camphylidenmethylen-anilino) -1,3,5-triazin | (1) | 0,50 |
| | Arlacel 581 | (2) | 6,00 |
| | Paraffinöl dickflüssig (Art.-Nr. 7160) | (1) | 17,50 |
| | Bienenwachs, gebleicht (Art.-Nr. 11544) | (1) | 3,00 |
| | Miglyol 812 | (3) | 11,50 |
| | Dow Corning 200 (100 cs) | (4) | 2,00 |
| | Tocopherolacetat (Art.-Nr. 500952) | (1) | 0,50 |
| | | | |
| B | Glycerin (Art.-Nr. 4093) | (1) | 2,00 |
| | Magnesiumsulfat-Heptahydrat (Art.-Nr. 5882) | (1) | 0,70 |
| | Konservierungsmittel | | q.s |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) ICI, Essen
(3) Hüls Troisdorf AG, Witten
(4) Dow Corning, Düsseldorf

### Beispiel 9

| % | | | |
|---|---|---|---|
| Sonnenschutzcreme (W/O) | | | |
| A | 2,4,6-Tris(4-camphyliden-methylenphenoxy)-1,3,5-triazin | (1) | 0,50 |
| | Arlacel 581 | (2) | 6,00 |
| | Paraffinöl dickflüssig (Art.-Nr. 7160) | (1) | 17,50 |
| | Bienenwachs, gebleicht (Art.-Nr. 11544) | (1) | 3,00 |
| | Miglyol 812 | (3) | 11,50 |
| | Dow Corning 200 (100 cs) | (4) | 2,00 |
| | Tocopherolacetat (Art.-Nr. 500952) | (1) | 0,50 |
| B | Glycerin (Art.-Nr. 4093) | (1) | 2,00 |
| | Magnesiumsulfat-Heptahydrat (Art.-Nr. 5882) | (1) | 0,70 |
| | Konservierungsmittel | | q.s |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) ICI, Essen
(3) Hüls Troisdorf AG, Witten
(4) Dow Corning, Düsseldorf

### Beispiel 10

| % | | | |
|---|---|---|---|
| Sonnenschutzmilch (W/O) | | | |
| A | 2,4,6-Tris(4-camphyliden-methylen-anilino)-1,3,5-triazin | (1) | 0,50 |
| | Abil WE 09 | (2) | 5,00 |
| | Paraffinöl dünnflüssig (Art.-Nr. 7174) | (1) | 16,00 |
| B | Natriumchlorid (Art.-Nr. 6400) | (1) | 2,00 |
| | Glycerin (Art.-Nr. 4093) | (1) | 3,00 |
| | Konservierungsmittel | | q.s |
| | Wasser, demineralisiert | | ad. 100,00 |
| | | | |
| C | Parfümöl Delaila | (3) | 0,50 |

### Herstellung:

Phase A auf 75 °C, Phase B auf 80 °C erhitzen. Die Phase B wird langsam in Phase A eingerührt. Homogenisieren. Unter Rühren abkühlen und bei 40 °C Phase C zugeben.

### Bezugsquellen:

(1) E. Merck, Darmstadt
(2) Th. Goldschmidt, Essen
(3) Dragoco, Holzminden

### Beispiel 11

| % | | | |
|---|---|---|---|
| Sonnenschutzmilch (W/O) | | | |
| A | 2,4,6-Tris(4-camphyliden- | | |
| | methylen-phenoxy)-1,3,5-triazin | (1) | 0,50 |
| | Abil WE 09 | (2) | 5,00 |
| | Paraffinöl dünnflüssig (Art.-Nr. 7174) | (1) | 16,00 |
| B | Natriumchlorid (Art.-Nr. 6400) | (1) | 2,00 |
| | Glycerin (Art.-Nr. 4093) | (1) | 3,00 |
| | Konservierungsmittel | | q.s |
| | Wasser, demineralisiert | | ad. 100,00 |
| | | | |
| C | Parfümöl Delaila | (3) | 0,50 |

### Herstellung:

Phase A auf 75 °C, Phase B auf 80 °C erhitzen. Die Phase B wird langsam in Phase A eingerührt. Homogenisieren. Unter Rühren abkühlen und bei 40 °C Phase C zugeben.

### Bezugsquellen:

(1) E. Merck, Darmstadt
(2) Th. Goldschmidt, Essen
(3) Dragoco, Holzminden

### Beispiel 12

| % | | | |
|---|---|---|---|
| Sonnenschutzcreme (O/W) | | | |
| | | | |
| A | 2,4,6-Tris(4-camphyliden-methylen-anilino)-1,3,5-triazin | (1) | 0,50 |
| | Brij 72 | (2) | 1,00 |
| | Arlacel 165 | (2) | 5,00 |
| | Paraffinöl flüssig (Art.-Nr. 7162) | (1) | 15,00 |
| | Cetylalkohol (Art.-Nr. 989) | (1) | 2,50 |
| | Miglyol 812 | (3) | 4,00 |
| | Isopropylmyristat | (4) | 2,00 |
| | | | |
| B | Glycerin (Art.-Nr. 4093) | (1) | 4,00 |
| | Karion F flüssig (Art.-Nr. 2993) | (1) | 5,00 |
| | Konservierungsmittel | | q.s |
| | Wasser, demineralisiert | | ad 100,00 |
| | | | |
| C | Parfümöl Wimbledon | (5) | 0,40 |

### Herstellung:

Phase A auf 75 °C, Phase B auf 80 °C erhitzen. Die Phase B wird langsam in Phase A eingerührt. Homogenisieren. Unter Rühren abkühlen und bei 40 °C Phase C zugeben.

### Bezugsquellen:

(1) E. Merck, Darmstadt
(2) ICI, Essen
(3) Hüls Troisdorf AG, Witten
(4) Henkel, Düsseldorf
(5) Haarmann & Reimer, Holzminden

### Beispiel 13

| % | | | |
|---|---|---|---|
| Sonnenschutzcreme (O/W) | | | |
| A | 2,4,6-Tris(4-camphylidenmethylen-phenoxy)-1,3,5-triazin | (1) | 0,50 |
| | Brij 72 | (2) | 1,00 |
| | Arlacel 165 | (2) | 5,00 |
| | Paraffinöl flüssig (Art.-Nr. 7162) | (1) | 15,00 |
| | Cetylalkohol (Art.-Nr. 989) | (1) | 2,50 |
| | Miglyol 812 | (3) | 4,00 |
| | Isopropylmyristat | (4) | 2,00 |
| | | | |
| B | Glycerin (Art.-Nr. 4093) | (1) | 4,00 |
| | Karion F flüssig (Art.-Nr. 2993) | (1) | 5,00 |
| | Konservierungsmittel | | q.s |
| | Wasser, demineralisiert | | ad 100,00 |
| | | | |
| C | Parfümöl Wimbledon | (5) | 0,40 |

### Herstellung:

Phase A auf 75 °C, Phase B auf 80 °C erhitzen. Die Phase B wird langsam in Phase A eingerührt. Homogenisieren. Unter Rühren abkühlen und bei 40 °C Phase C zugeben.

### Bezuqsquellen:

(1) E. Merck, Darmstadt
(2) ICI, Essen
(3) Hüls Troisdorf AG, Witten
(4) Henkel, Düsseldorf
(5) Haarmann & Reimer, Holzminden

## Patentansprüche

1. Triazin-Derivate der Formel I 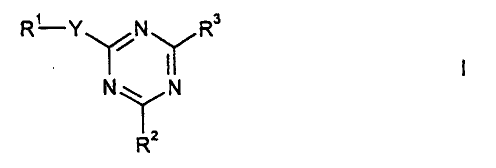 wobei
Y NH oder O
R¹ einen Rest der Formel II 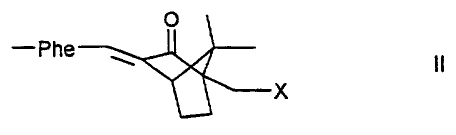
worin
Phe eine unsubstituierte oder durch 1 bis 4 Alkyl- oder Alkoxygruppen mit 1 bis 10 C-Atomen substituierte Phenylengruppe
und
X H oder -SO₃H
bedeuten, und
R² und R³ jeweils unabhängig voneinander einen Rest der Formel Y-R¹
oder
Alkoxy, Alkylamino mit 1 bis 10 C-Atomen,
Arylamino oder Aryloxy mit bis zu 10 C-Atomen
bedeuten,
wobei jedoch Verbindungen ausgenommen sind, in denen jeweils zwei der Triazin-Substituenten Y-R¹, R² oder R³ 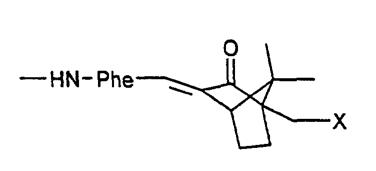 mit
Phe unsubstituierte Phenylengruppe
X H
und der dritte Triazin-Substituent
Alkoxy oder Alkylamino mit 8 bis 10 C-Atomen
bedeuten.

2. Triazin-Derivate der Formel 11 nach Anspruch 1 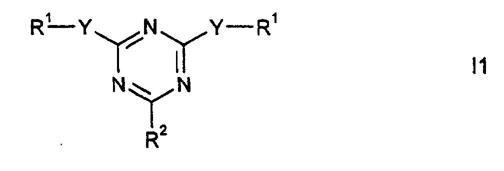 worin R¹, R² und Y die angegebene Bedeutung besitzen,
wobei jedoch Verbindungen ausgenommen sind, in denen
Y-R¹ 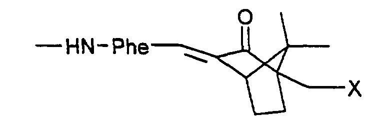 mit
Phe unsubstituierte Phenylengruppe
X H
und
R² Alkoxy oder Alkylamino mit 8 bis 10 C-Atomen
bedeuten.

3. Triazin-Derivate der Formel 12 nach Anspruch 1 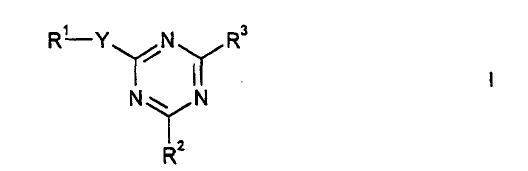 worin
R¹ und Y die angegebene Bedeutung besitzen und
R² und R³ jeweils unabhängig voneinander Alkoxy, Alkylamino mit 1 bis 10 C-Atomen, Arylamino oder Aryloxy mit bis zu 10 C-Atomen
bedeuten.

4. Triazin-Derivat nach Anspruch 1 oder 2, der Formel la 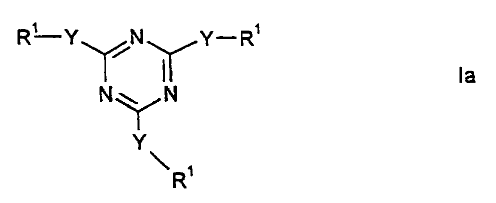 worin Phe eine unsubstituierte 1,4-Phenylengruppe und X H bedeuten.

5. Triazin-Derivat nach einem der Ansprüche 1 bis 4, worin Y NH bedeutet.

6. Triazin-Derivat nach Anspruch 3 der Formel Ic 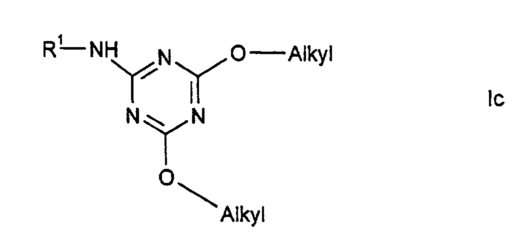 worin R¹ die angegebene Bedeutung besitzt, und Alkyl Alkyl mit 1 bis 10 C-Atomen bedeutet.

7. Triazin-Derivat nach Anspruch 3 der Formel Id 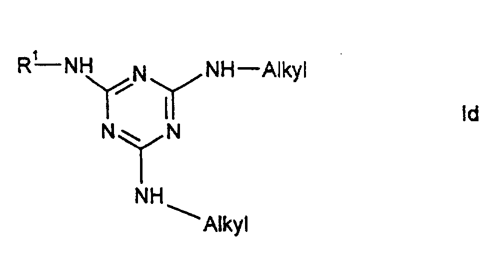 worin R¹ die angegebene Bedeutung besitzt, und Alkyl Alkyl mit 1 bis 10 C-Atomen bedeutet.

8. Verfahren zur Herstellung der Verbindungen der Formel Ic, **dadurch gekennzeichnet, daß** man bei Temperaturen zwischen 0°C und Raumtemperatur äquimolare Mengen an Cyanurchlorid und einer Verbindung der Formel Ila,
R¹NH₂ IIa
worin R¹ die angegebene Bedeutung besitzt, gegebenenfalls in Gegenwart einer schwachen Base, miteinander umsetzt, und das dabei entstehende Triazin-Derivat der Formel llla, 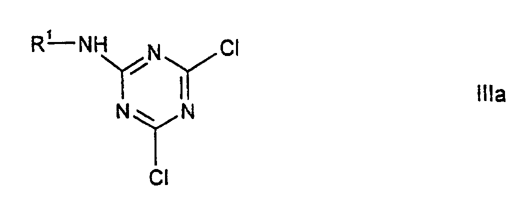 worin R¹ die angegebene Bedeutung besitzt, isoliert und
anschließend mit mindestens 2 Mol eines Alkohols umsetzt.

9. Verbindung der Formel IIIa 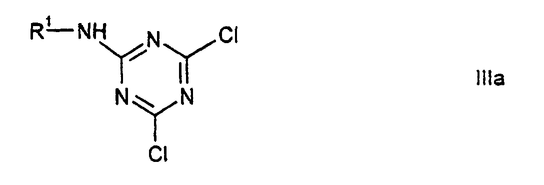 worin R¹ die Bedeutung
eines Rests der Formel II 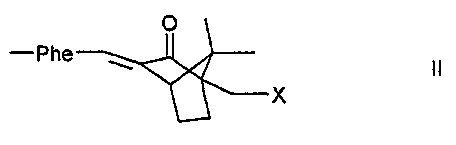 worin
Phe eine unsubstituierte oder durch 1 bis 4 Alkyl- oder Alkoxygruppen mit 1 bis 10 C-Atomen substituierte Phenylengruppe
und
X H oder -SO₃H
bedeuten,
besitzt.

10. Verwendung der Verbindungen der Formel I nach einem der Ansprüche 1 bis 7 als Sonnenfilter im Wellenlängenbereich von 280 bis 400 nm.

11. Kosmetische Zubereitung, **dadurch gekennzeichnet, daß** sie eine wirksame Menge mindestens einer Verbindung der Formel I nach Anspruch 1 in einem kosmetisch verträglichen Träger enthält.

12. Kosmetische Zubereitung nach Anspruch 11, wobei der Träger mindestens eine Fettphase aufweist, **dadurch gekennzeichnet, daß** sie mindestens eine Verbindung der Formeln Ia, Ib, Ic, oder Id enthält.

13. Kosmetische Zubereitung nach einem der Ansprüche 11 bis 12, **dadurch gekennzeichnet, daß** sie 0.1 bis 10 Gew.-%, vorzugsweise 0.4 bis 1.0 Gew.-%, insbesondere ca. 0.5 Gew.-% mindestens einer Verbindung der Formel I enthält.

14. Verwendung der Verbindungen der Formel I nach einem der Ansprüche 1 bis 7 zur Herstellung eines Medikaments.

15. Verwendung gemäß Anspruch 14 zur Herstellung eines Medikaments zur vorbeugenden Behandlung der Haut gegen Entzündungen und Allergien.

## Claims

1. Triazine derivatives of the formula I 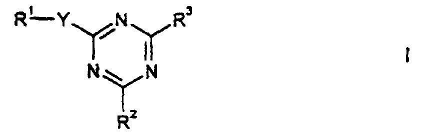 wherein
Y is NH or O,
R¹ is a radical of the formula II 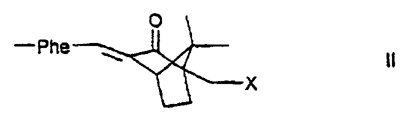
wherein
Phe is a phenylene group which is unsubstituted or substituted by 1 to 4 alkyl or alkoxy groups having 1 to 10 C atoms
and
X is H or -SO₃H,
and
R² and R³ in each case independently of one another are a radical of the formula Y-R¹,
or
alkoxy, alkylamino having 1 to 10 C atoms, arylamino or aryloxy having up to 10 C atoms,
but excepting compounds in each of which two of the triazine substituents Y-R¹, R² or R³ are 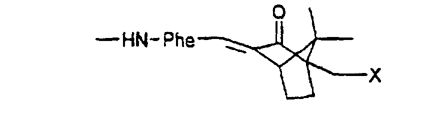
Phe is unsubstituted phenylene group
X is H
and the third triazine substituent is alkoxy or alkylamino having 8 to 10 C atoms.

2. Triazine derivatives of the formula I1 according to Claim 1 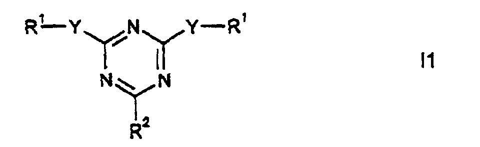 wherein R¹, R² and Y have the meaning given,
but excepting compounds in which
Y-R¹ is 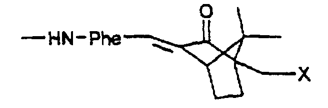 where
Phe is unsubstituted phenylene group
X is H
and
R2 is alkoxy or alkylamino having 8 to 10 C atoms.

3. Triazine derivatives of the formula I2 according to Claim 1 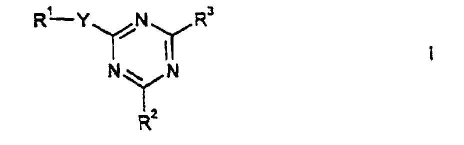 wherein
R¹ and Y have the meaning given and
R² and R³ in each case independently of one another are alkoxy, alkylamino having 1 to 10 C atoms, arylamino or aryloxy having up to 10 C atoms.

4. Triazine derivative according to Claim 1 or 2, of the formula Ia 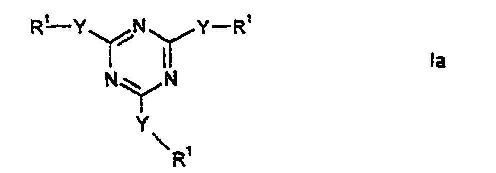 wherein Phe is an unsubstituted 1,4-phenylene group and X is H.

5. Triazine derivative according to one of Claims 1 to 4, wherein Y is NH.

6. Triazine derivative according to Claim 3 of the formula Ic 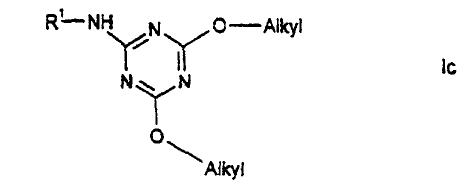 wherein R¹ has the meaning given and Alkyl is alkyl having 1 to 10 C atoms.

7. Triazine derivative according to Claim 3 of the formula Id 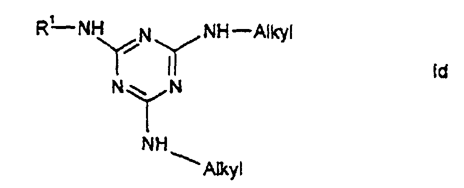 wherein R¹ has the meaning given and Alkyl is alkyl having 1 to 10 C atoms.

8. Process for the preparation of the compounds of the formula Ic, **characterized in that** equimolar amounts of cyanuric chloride and a compound of the formula IIa
R¹NH₂ IIa
wherein R¹ has the meaning given, are reacted with one another at temperatures between 0°C and room temperature, if appropriate in the presence of a weak base, and the triazine derivative thereby formed, of formula IIIa 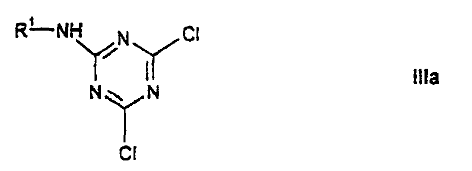 wherein R¹ has the meaning given, is isolated and then reacted with at least 2 mol of an alcohol.

9. Compound of the formula IIIa 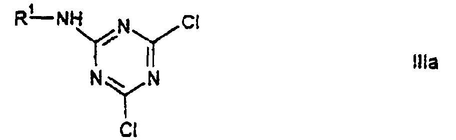 wherein R¹ has the meaning of a radical of the formula II 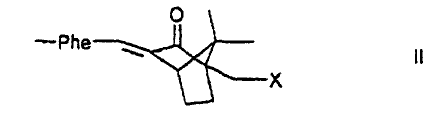 wherein
Phe is a phenylene group which is unsubstituted or substituted by 1 to 4 alkyl or alkoxy groups having 1 to 10 C atoms
and
X is H or -SO₃H,

10. Use of the compounds of the formula I according to one of Claims 1 to 7 as a sun filter in the wavelength range from 280 to 400 nm.

11. Cosmetic formulation, **characterized in that** it comprises an active amount of at least one compound of the formula I according to Claim 1 in a cosmetically tolerated carrier.

12. Cosmetic formulation according to Claim 11, wherein the carrier contains at least one fatty phase, **characterized in that** it comprises at least one compound of the formulae Ia, Ib, Ic or Id.

13. Cosmetic formulation according to one of Claims 11 to 12, **characterized in that** it comprises 0.1 to 10% by weight, preferably 0.4 to 1.0% by weight, in particular about 0.5% by weight, of at least one compound of the formula I.

14. Use of the compounds of the formula I according to one of Claims 1 to 7 for producing a medicament.

15. Use according to Claim 14 for producing a medicament for the preventive treatment of the skin against inflammations and allergies.

## Revendications

1. Dérivés de triazine de formule I 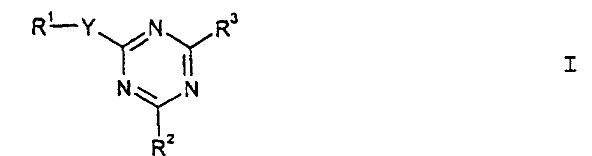 dans laquelle
Y est NH ou O,
R¹ est un radical de formule II 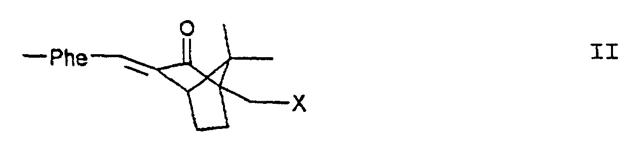
dans laquelle
Phe est un groupe phénylène non-substitué ou substitué par 1 à 4 groupes alkyle ou alcoxy ayant de 1 à 10 atomes de carbone,
et
X est H ou -SO₃H,
et
R² et R³ représentent chacun indépendamment de l'autre un radical de formule Y-R¹, ou encore alcoxy, alkylamino ayant de 1 à 10 atomes de carbone, arylamino ou aryloxy ayant jusqu'à 10 atomes de carbone,
mais cependant à l'exception des composés dans lesquels deux des substituants Y-R¹, R² ou R³ de la triazine représentent 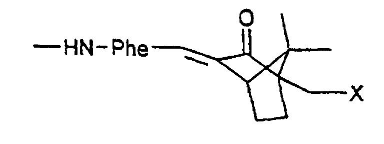 où
Phe est un groupe phénylène non-substitué,
X est H,
et le troisième substituant de la triazine est un groupe alcoxy ou alkylamino ayant de 8 à 10 atomes de carbone.

2. Dérivés de triazine de formule I1 selon la revendication 1 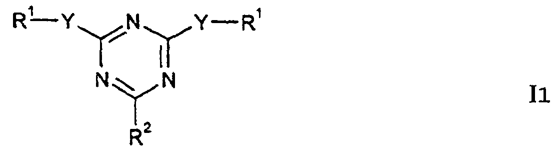 dans laquelle R¹, R² et Y ont les significations données,
mais cependant à l'exception des composés dans lesquels
Y-R¹ est 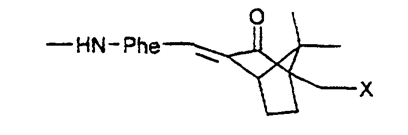 où
Phe est un groupe phénylène non-substitué
X est H,
et
R² est un groupe alcoxy ou alkylamino ayant de 8 à 10 atomes de carbone.

3. Dérivés de triazine de formule I2 selon la revendication 1 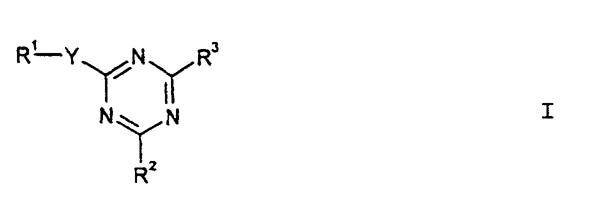 dans laquelle
R¹ et Y ont les significations données, et
R² et R³ représentent chacun indépendamment de l'autre un groupe alcoxy, alkylamino ayant de 1 à 10 atomes de carbone, arylamino ou aryloxy ayant jusqu'à 10 atomes de carbone.

4. Dérivé de triazine selon la revendication 1 ou 2, de formule Ia 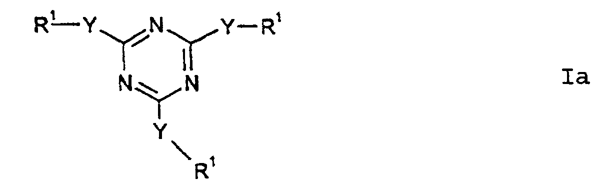 dans laquelle Phe est un groupe 1,4-phénylène non-substitué, et X est H.

5. Dérivé de triazine selon l'une des revendications 1 à 4, dans lequel Y est NH.

6. Dérivé de triazine selon la revendication 3, de formule Ic 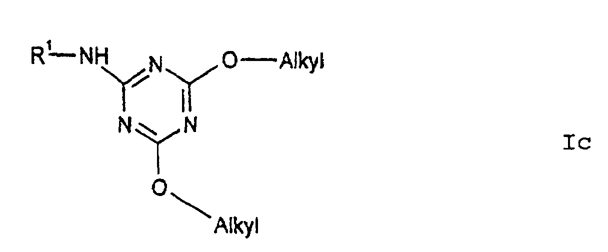 dans laquelle R¹ a les significations données, et alkyl représente un groupe alkyle ayant de 1 à 10 atomes de carbone.

7. Dérivé de triazine selon la revendication 3, de formule Id 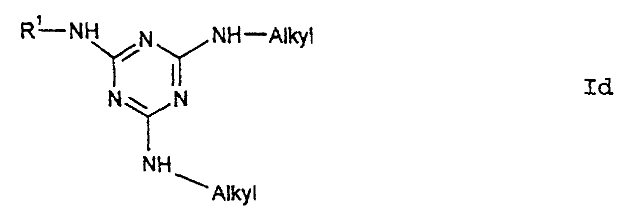 dans laquelle R¹ a les significations données, et alkyl représente un groupe alkyle ayant de 1 à 10 atomes de carbone.

8. Procédé de préparation des composés de formule Ic, **caractérisé en ce que**, à des températures comprises entre 0°C et la température ambiante, on fait réagir l'un avec l'autre des quantités équimolaires de chlorure de cyanuryle et d'un composé de formule IIa
R¹NH₂ IIa
dans laquelle R¹ a les significations données, éventuellement en présence d'une base faible, et en isole le dérivé de triazine de formule IIIa qui se forme à cette occasion 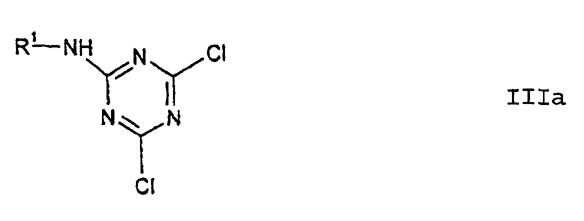 dans laquelle R¹ a les significations données, puis on le fait réagir avec au moins 2 moles d'un alcool.

9. Composé de formule IIIa 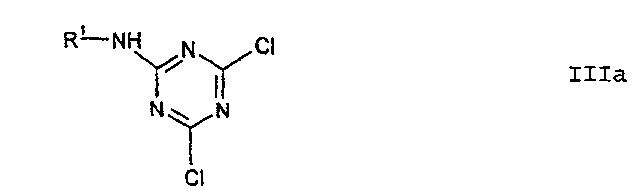 dans laquelle R¹ représente un radical de formule II 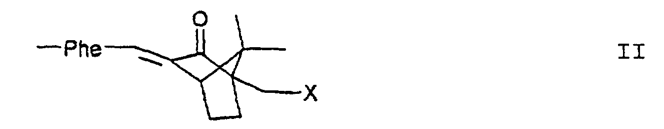 dans laquelle
Phe est un groupe phénylène non-substitué ou substitué par 1 à 4 groupes alkyle ou alcoxy ayant de 1 à 10 atomes de carbone,
et
X est H ou -SO₃H.

10. Utilisation des composés de formule I selon l'une des revendications 1 à 7 en tant que filtre solaire dans la plage de longueurs d'ondes de 280 à 400 nm.

11. Préparation cosmétique, **caractérisée en ce qu'**elle contient une quantité efficace d'au moins un composé de formule I selon la revendication 1 dans un support toléré du point de vue cosmétique.

12. Préparation cosmétique selon la revendication 11, dans laquelle le support comporte au moins une phase grasse, **caractérisée en ce qu'**elle contient au moins un composé ayant les formules Ia, Ib, Ic ou Id.

13. Préparation cosmétique selon l'une des revendications 11 à 12, **caractérisée en ce qu'**elle contient de 0,1 à 10 % en poids, de préférence de 0,4 à 1,0 % en poids, en particulier environ 0,5 % en poids d'au moins un composé de formule I.

14. Utilisation des composés de formule I selon l'une des revendications 1 à 7 pour préparer un médicament.

15. Utilisation selon la revendication 14 pour préparer un médicament destiné au traitement préventif de la peau contre les inflammations et les allergies.
